# EUROPÄISCHE PATENTSCHRIFT

(11) **EP 0 691 127 B1**
(45) Veröffentlichungstag und Bekanntmachung des Hinweises auf die Patenterteilung: **25.10.2000**
(21) Anmeldenummer: 95109370.7
(22) Anmeldetag: 16.06.1995
(51) Int. Cl.: A61K 7/50

(54) **Duschöl**
Shower oil
Huile de douche

(30) Priorität: 09.07.1994 DE 4424210
(43) Veröffentlichungstag der Anmeldung: 10.01.1996
(73) Patentinhaber: Beiersdorf Aktiengesellschaft, 20245 Hamburg (DE)
(72) Erfinder: Gerber, Bozena, D-22529 Hamburg (DE); Schmidt-Lewerkühne, Hartmut, Dr., D-22869 Schenenfeld (DE); Stelling, Otto, D-22529 Hamburg (DE); Schmucker, Robert, Dr., D-22457 Hamburg (DE)

(56) Entgegenhaltungen:
- AT-B- 279 049
- DE-A- 1 467 963
- DE-A- 2 943 202
- DE-A- 3 413 563

## Beschreibung

Die vorliegende Erfindung betrifft kosmetische Reinigungszubereitungen, bevorzugt für die Verwendung als Duschpräparat.

Derartige Zubereitungen sind an sich bekannt. Es handelt sich dabei im wesentlichen um oberflächenaktive Substanzen oder Stoffgemische, die dem Verbraucher in verschiedenen Zubereitungen angeboten werden. Zubereitungen solcher Art zeichnen sich im allgemeinen durch einen mehr oder weniger hohen Wassergehalt aus, können aber auch beispielsweise als Konzentrat vorliegen.

Im allgemeinen unterscheiden sich Präparate, welche für das Duschbad vorgesehen sind, nicht oder kaum von Wannenbadzubereitungen, abgesehen davon, daß bei Duschzubereitungen Produkte höherer Viskosität bevorzugt werden, die nicht nach Entnahme aus dem Behälter aus der Hand rinnen. Dies ist bei Wannenbadzubereitungen weniger von praktischer Bedeutung.

Schon bei einem einfachen Wasserbade ohne Zusatz von Tensiden kommt es zunächst zu einer Quellung der Homschicht der Haut, wobei der Grad dieser Quellung beispielsweise von der Dauer des Bades und dessen Temperatur abhängt. Zugleich werden wasserlösliche Stoffe, z.B. wasserlösliche Schmutzbestandteile, aber auch hauteigene Stoffe, die für das Wasserbindungsvermögen der Homschicht verantwortlich sind, ab-bzw. ausgewaschen. Durch hauteigene oberflächenaktive Stoffe werden zudem auch Hautfette in gewissem Ausmaße gelöst und ausgewaschen. Dies bedingt nach anfänglicher Quellung eine nachfolgende deutliche Austrocknung der Haut, die durch waschaktive Zusätze nach verstärkt werden kann.

Bei gesunder Haut sind diese Vorgänge im allgemeinen belanglos, da die Schutzmechanismen der Haut solche leichten Störungen der oberen Hautschichten ohne weiteres kompensieren können. Aber bereits im Falle nichtpathologischer Abweichungen vom Normalstatus, z.B. durch umweltbedingte Abnutzungsschäden bzw. Irritationen, Lichtschäden, Altershaut usw., ist der Schutzmechanismus der Hautoberfläche gestört. Unter Umständen ist er dann aus eigener Kraft nicht mehr imstande, seine Aufgabe zu erfüllen und muß durch externe Maßnahmen regeneriert werden.

Der Stand der Technik kennt Ölbadzubereitungen verschiedener Art, wobei die Eigenschaften der Fett- oder Ölphase durch Zugabe von oberflächenaktiven Substanzen variiert werden kann. Dabei können je nach Art und Menge der gewählten Bestandteile Zubereitungen formuliert werden, die auf der Badewasseroberfläche entweder spreitende Ölfilme, Öl-in-Wasser-Systeme oder auch Totalsolubilisate ergeben. Schäumende, aber auch wenig schäumende oder nicht schäumende Formulierungen sind möglich.

Im allgemeinen beschränkt sich die Funktionalität derartiger Zubereitungen bei Ölbad- oder Ölcrèmebadzubereitungen auf die Rückfettung oder Überfettung der obersten Hautschichten. Die Europäische Offenlegungsschrift 120 224 beschreibt allerdings wirkstoffhaltige Ölbadzubereitungen mit einem Gehalt an 38,75 Gew.-% Sojaöl, 2,00 Gew.-% Rizinusöl, 37,00 Gew.-% Vaselineöl. Als Emulgator werden Polyethylenglycolmonobzw. -diester offenbart, in Konzentrationen von ca. 10 - 12 Gew.-%.

Ein erheblicher Nachteil des Standes der Technik ist, daß derartige Badezubereitungen in sehr großer Verdünnung (Inhalt einer Badewanne: im Einzelfalle mehrere hundert Liter Wasser) vorliegen. Dem muß durch besondere Formulierungssorgfalt bzw. Anwendung großer Mengen der zu verwendenden Ölbadzubereitung Rechnung getragen werden.

Dem Stande der Technik unbekannt sind tensidhaltige Duschzubereitungen mit hohem Ölgehalt, welche als Duschöle bezeichnet werden könnten, wohl in erster Linie deshalb, weil der Fachmann annehmen mußte, daß Zubereitungen mit hohem Ölgehalt praktisch keine Schaumwirkung - oder aber keine Pflegewirkung entfalten würden.

Die nicht anwendungsgerechter Nutzung einer Ölschaumbadzubereitung als Duschzubereitung ist da keine Alternative. Diese Nutzung ist unzweckmäßig, da solche Zubereitungen keinen nennenswerten Schaum entwickeln.

Aufgabe der vorliegenden Erfindung war somit, diesem Mangel des Standes der Technik Abhilfe zu schaffen. Weiterhin war eine Aufgabe der Erfindung, Duschbadzubereitungen zur Verfügung zu stellen, welche einesteils hohe Pflegewirkung besitzen, ohne daß andererseits die reinigende Wirkung dahinter zurücksteht.

Erstaunlicherweise stellte sich heraus, daß die Verwendung von Zusammensetzungen mit
(a) einem Gehalt von höchstens 55 Gew.-%, bezogen auf das Gesamtgewicht der Zubereitungen, eines oder mehrerer Tenside, gewählt aus der Gruppe Fettalkoholethoxylate, Fettalkoholsulfate, Amide der Fettalkoholsulfate, Fettalkoholethersulfate, Amide der Fettalkoholethersulfate, Fettsäuremonoethanolamide, Fettsäurediethanolamide, sowie
(b) einem Gehalt von mindestens 45 Gew.-%, bezogen auf das Gesamtgewicht der Zubereitungen, einer oder mehrerer Ölkomponenten, gewählt aus der Gruppe der Öle mit einem hohen Gehalt an Triglyceriden gesättigter und/oder ungesättigter, verzweigter und/oder unverzweigter Fettsäuren oder ausschließlich solche Triglyceride enthaltend,
(c) die im übrigen im wesentlichen wasserfrei sind,
zur Herstellung kosmetischer oder dermatologischer Duschöle
die der Erfindung zugrundeliegenden Aufgaben in höchst zufriedenstellender Weise erfüllen. Die erfindungsgemäßen Zubereitungen haben sehr gute Schaumentwicklung, hohe Reinigungskraft und wirken in hohem Maße regenerierend in bezug auf den allgemeinen Hautzustand. Insbesondere wirken die erfindungsgemäßen Zubereitungen hautglättend, vermindern das Trockenheitsgefühl der Haut und machen die Haut geschmeidig.

Zwar werden in den Druckschriften DE-A-34 13 563, DE-A-29 43 202, DE-A-14 67 963, AT-B-0 279 049, US-A-4,371,548, US-A-4,707,293 und DE-C-27 00 891 Dusch- und Badezubereitungen beschrieben, die aber den Weg zur vorliegenden Erfindung nicht weisen konnten.

Vorteilhaft weisen die erfindungsgemäß günstig zu verwendenden Fettalkoholsulfate bzw. Fettalkoholethersulfate folgende Struktur auf: 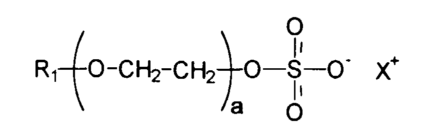 Dabei kann a Werte von 0 bis 10, vorteilhaft 1 bis 5 annehmen. R¹ wird gewählt aus der Gruppe der verzweigten und unverzweigten Alkylgruppen mit 6 bis 24 Kohlenstoffatomen.

X⁺ wird gewählt aus der Gruppe der Alkaliionen sowie der Gruppe der mit einer oder mehreren Alkyl- und/oder mit einer oder mehreren Hydroxyalkylresten substituierten Ammoniumionen.

Vorteilhaft weisen die erfindungsgemäß günstig zu verwendenden Amide der Fettalkoholsulfate bzw. der Fettalkoholethersulfate folgende Struktur auf: 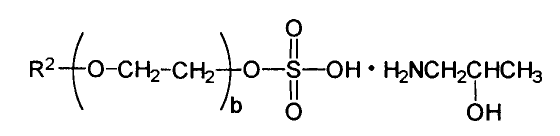 Dabei kann b Werte von 0 bis 10, vorteilhaft 1 bis 5 annehmen. R² wird gewählt aus der Gruppe der verzweigten und unverzweigten Alkylgruppen mit 6 bis 24 Kohlenstoffatomen.

Bevorzugtes Fettalkoholethersulfat ist MIPA-Laurethsulfat.

Vorteilhaft weisen die erfindungsgemäß günstig zu verwendenden Fettalkoholethoxylate folgende Struktur auf: 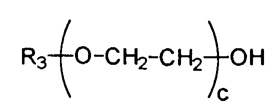 Dabei kann c Werte von 1 bis 45 annehmen, bevorzugt von 1 bis 10. R³ wird gewählt aus der Gruppe der verzweigten und unverzweigten Alkylgruppen mit 6 bis 24 Kohlenstoffatomen.

Bevorzugtes Fettalkoholethoxylat ist Laureth-4.

Vorteilhaft weisen die erfindungsgemäß günstig zu verwendenden Fettsäuremono- bzw. -diethanolamide folgende Strukturen auf: 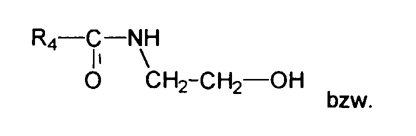 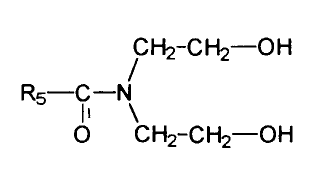 R⁴ bzw. R⁵ werden dabei gewählt aus der Gruppe der verzweigten und unverzweigten Alkylgruppen und/oder Alkenylgruppen mit 6 bis 24 Kohlenstoffatomen.

Bevorzugtes Fettsäurediethanolamid ist Kokosfettsäurediethanolamid (Cocamide DEA). Natürliche Kokosfettsäure enthält als wesentliche Bestandteile Laurinsäure zu 44 - 51 Gew.-%, Myristinsäure zu 13 - 18 Gew.-%, Palmitinsäure zu 8- 10 Gew.-%, Caprylsäure zu 6 - 9 Gew.-%, Caprinsäure zu 6 - 10 Gew.-%, Ölsäure zu 5 - 8 Gew.-%, Stearinsäure zu 1 - 3 Gew.-%, Linolsäure zu 0 - 2 Gew.-% und Capronsäure zu 0 - 1 Gew.-%.

Ganz besonders bevorzugt ist, Gemische aus MIPA-Laurethsulfat, Laureth-4 und Kokosfettsäurediethanolamid einzusetzen. Solche Gemische sind beispielsweise unter der Bezeichnung ZETESOL® 100 von der Firma Zschimmer & Schwarz Chemische Fabriken, Lahnstein/Rhein, oder TEXAPON® WW 99 von der Henkel KGaA, Düsseldorf, erhältlich.

Die erfindungsgemäßen Öle werden vorzugsweise gewählt aus der Gruppe der Triglyceride folgender Struktur: 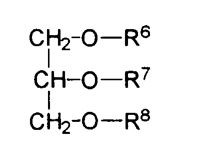 wobei R⁶, R⁷ und R⁸ unabhängig voneinander gewählt werden aus der Gruppe der verzweigten und unverzweigten, Alkylcarboxyl- bzw. Alkenylcarboxylgruppen mit 12 bis 24 Kohlenstoffatomen. Es ist gegebenenfalls vorteilhaft, wenn eine oder mehrere aliphatische Wasserstoffatome der Alkylcarboxyl- bzw. Alkenylcarboxylgruppen durch Hydroxylgruppen substituiert sind.

Insbesondere ist vorteilhaft, wenn R⁶, R⁷ und/oder R⁸ 16 bis 20 Kohlenstoffatome aufweisen und aus der Gruppe der einfach bis dreifach ungesättigten Carbonsäurereste gewählt werden.

Wenn R⁶, R⁷ und/oder R⁸ Hydroxylgruppen tragen, ist der bevorzugte Alkenylcarboxylrest der Ricinolsäurerest.

Besonders vorteilhaft ist, die erfindungsgemäßen Öle aus der Gruppe Sojaöl, Sonnenblumenöl, Weizenkeimöl und Ricinusöl zu wählen.

Bevorzugte Zubereitungen enthalten 0 bis 60 Gew.-% Sojaöl und 0 bis 60 Gew.-% Weizenkeimöl und 0 bis 60 Gew.-% Sonnenblumenöl, mit der Maßgabe, daß die Summe der Einzelkonzentrationen dieser Öle 30 - 60 Gew.-% ausmacht, ferner Rizinusöl in einer Konzentration von 5 - 25 Gew.-%, wobei diese Konzentrationen jeweils bezogen sind auf das Gesamtgewicht der Zubereitungen.

Zwar sind aus der Firmenschrift "ZETESOL 100" Rahmenrezepturen bekannt, welche neben dem erfindungsgemäß vorteilhaft zu verwendenden Tensidgemisch Zetesol 100 auch Sonnenblumenöl, Weizenkeimöl bzw. Sojaöl enthalten. Die eine Rezeptur ist zwar wasserfrei, enthält aber 72 Gew.-% Tensidgemisch und nur 25 Gew.-% Öle. Die andere Rezeptur enthält 5 Gew.-% Wasser, 68 Gew.-% Tensidgemisch und nur 15 Gew.-% Öle.

Es handelt sich ferner bei den Zubereitungen des Standes der Technik um Ölschaumbäder mit den geschilderten Nachteilen. Die Ölkonzentration wird am angegebenen Orte bereits als "sehr hoch" geschildert. Der Stand der Technik hat also angenommen, daß eine weitere Erhöhung der Ölkonzentration zumindest problematisch sein würde. Dieses Vorurteil beseitigt die vorliegende Erfindung.

Besonders überraschend war, daß die erfindungsgemäßen Zubereitungen, trotz des hohen Ölanteils, bei der Anwendung einen reichen, äußerst feinblasigen, cremigen Schaum ergeben.

Die erfindungsgemäßen Zusammensetzungen enthalten außer den vorgenannten Tensiden Wasser und gegebenenfalls die in der Kosmetik üblichen Zusatzstoffe, beispielsweise Parfüm, Verdicker, Lösungsvermittler, Farbstoffe, Desodorantien, antimikrobielle Stoffe, rückfettende Agentien, Komplexierungs- und Sequestrierungsagentien, Perlglanzagentien, Pflanzenextrakte, Vitamine, Wirkstoffe und dergleichen.

Vorteilhaft ist insbesondere, Lösungsvermittler aus der Gruppe der Polyoxyethylen-Polyoxypropylen-Blockcopolymere zu wählen. Solche Blockcoploymere sind unter der Bezeichnung "Poloxamere" bekannt und zeichnen sich durch folgende Struktur aus: 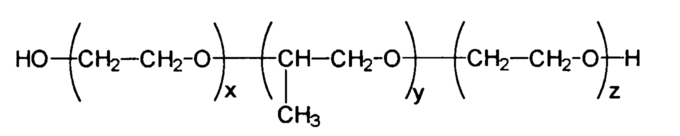 Dabei nimmt x vorteilhaft Werte zwischen 2 und 20 an. y nimmt vorteilhaft Werte zwischen 10 und 50 an. z nimmt vorteilhaft Werte zwischen 2 und 20 an.

Wenn Zubereitungen gemäß der vorliegenden Erfindung außer den erfindungsgemäßen Tensiden weitere Tenside enthalten sollen, so wird bevorzugt, deren Konzentration in bezug auf das Gewicht der Gesamtzusammensetzung nicht größer als 5 Gew.-% zu wählen.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe

Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Carnosin und deren Derivate (z.B. Anserin), Carotinoide, Carotine (z.B.α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl, Butyl- und Lauryl-, Palmitoyl-, Oleyl-, γ-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Pentat-, Hexa-, Heptahioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubiquinon und Ubiquinol deren Derivate, Vitamin C und Derivate (z.B. Ascorbylpalmitate, Mg - Ascorbylphosphate, Ascorbylacetate), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können öllösliche Antioxidantien eingesetzt werden.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die nachfolgenden Beispiele sollen die vorliegende Erfindung verdeutlichen. Die Zahlenwerte in den Beispielen bedeuten Gewichtsprozente, bezogen auf das Gesamtgewicht der jeweiligen Zubereitungen.

| Beispiel | 1 | 2 | 3 |
|---|---|---|---|
| Sojaöl | 54 | 37 | 42 |
| Rizinusöl | 14 | 9 | 14 |
| ZETESOL®100 | 30 | 51 | 41 |
| Poloxamer 101 | 2 | 2 | 2 |
| Parfum, Antioxidantien, Konservierungsstoffe | q.s. | q.s. | q.s. |

| Beispiel | 4 | 5 | 6 |
|---|---|---|---|
| Weizenkeimöl | 54 | 37 | 42 |
| Rizinusöl | 14 | 9 | 14 |
| ZETESOL®100 | 30 | 51 | 41 |
| Poloxamer 101 | 2 | 2 | 2 |
| Parfum, Antioxidantien, Konservierungsstoffe | q.s. | q.s. | q.s. |

| Beispiel | 7 | 8 | 9 |
|---|---|---|---|
| Sonnenblumenöl | 54 | 37 | 42 |
| Rizinusöl | 14 | 9 | 14 |
| ZETESOL®100 | 30 | 51 | 41 |
| Poloxamer 101 | 2 | 2 | 2 |
| Parfum, Antioxidantien, Konservierungsstoffe | q.s. | q.s. | q.s. |

## Patentansprüche

1. Verwendung von Zusammensetzungen mit
(a) einem Gehalt von höchstens 55 Gew.-%, bezogen auf das Gesamtgewicht der Zubereitungen, eines oder mehrerer Tenside, gewählt aus der Gruppe Fettalkoholethoxylate, Fettalkoholsulfate, Amide der Fettalkoholsulfate, Fettalkoholethersulfate, Amide der Fettalkoholethersulfate, Fettsäuremonoethanolamide, Fettsäurediethanolamide, sowie
(b) einem Gehalt von mindestens 45 Gew.-%, bezogen auf das Gesamtgewicht der Zubereitungen, einer oder mehrerer Ölkomponenten, gewählt aus der Gruppe der Öle mit einem hohen Gehalt an Triglyceriden gesättigter und/oder ungesättigter, verzweigter und/oder unverzweigter Fettsäuren oder ausschließlich solche Triglyceride enthaltend,
(c) die im übrigen im wesentlichen wasserfrei sind,
zur Herstellung kosmetischer oder dermatologischer Duschöle.

2. Verwendung nach Anspruch 1, dadurch gekennzeichnet, daß zusätzlich weitere Tenside und/oder kosmetische oder pharmazeutische Hilfs-, Zusatz- und/oder Wirkstoffe enthalten sind, die den Gehalt an (a) entsprechend vermindern.

3. Verwendung nach Anspruch 1, dadurch gekennzeichnet, daß die Fettalkoholsulfate bzw. Fettalkoholethersulfate folgende Struktur aufweisen: 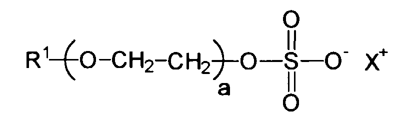 wobei a Werte von 0 bis 10, vorteilhaft 1 bis 5, annimmt und R¹ gewählt wird aus der Gruppe der verzweigten und unverzweigten Alkylgruppen mit 6 bis 24 Kohlenstoffatomen und X⁺ gewählt wird aus der Gruppe der Alkaliionen sowie der Gruppe der mit einer oder mehreren Alkyl- und/oder mit einer oder mehreren Hydroxyalkylresten substituierten Ammoniumionen.

4. Verwendung nach Anspruch 1, dadurch gekennzeichnet, daß die Amide der Fettalkoholsulfate bzw. der Fettalkoholethersulfate folgende Struktur aufweisen: 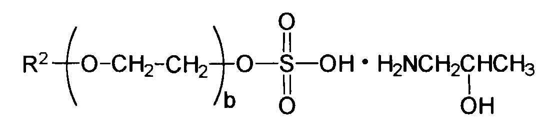 wobei b Werte von 0 bis 10, vorteilhaft 1 bis 5 annimmt und R² gewählt wird aus der Gruppe der verzweigten und unverzweigten Alkylgruppen mit 6 bis 24 Kohlenstoffatomen.

5. Verwendung nach Anspruch 1, dadurch gekennzeichnet, daß die Fettalkoholethoxylate folgende Struktur aufweisen: 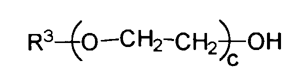 wobei c Werte von 1 bis 45 annimmt, bevorzugt von 1 bis 10 und R³ gewählt wird aus der Gruppe der verzweigten und unverzweigten Alkylgruppen mit 6 bis 24 Kohlenstoffatomen.

6. Verwendung nach Anspruch 1, dadurch gekennzeichnet, daß die Fettsäuremono- bzw. -diethanolamide folgende Strukturen aufweisen: 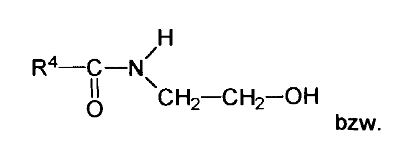 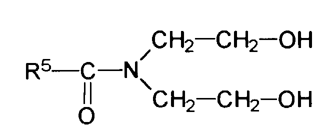 wobei R⁴ bzw. R⁵ gewählt werden aus der Gruppe der verzweigten und unverzweigten Alkylgruppen und/oder Alkenylgruppen mit 6 bis 24 Kohlenstoffatomen.

7. Verwendung nach Anspruch 1, dadurch gekennzeichnet, daß die Öle gewählt werden aus der Gruppe der Triglyceride folgender Struktur: 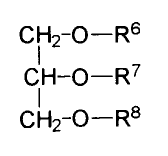 wobei R⁶, R⁷ und R⁸ unabhängig voneinander gewählt werden aus der Gruppe der verzweigten und unverzweigten, Alkylcarboxyl- bzw. Alkenylcarboxylgruppen mit 12 bis 24 Kohlenstoffatomen und gegebenenfalls ein oder mehrere Wasserstoffatome der Alkylcarboxyl- bzw. Alkenylcarboxylgruppen durch Hydroxylgruppen substituiert sind.

8. Verwendung nach Anspruch 7, dadurch gekennzeichnet, daß R⁶, R⁷ und/oder R⁸ 16 bis 20 Kohlenstoffatome aufweisen und aus der Gruppe der einfach bis dreifach ungesättigten Carbonsäurereste gewählt werden.

9. Verwendung nach Anspruch 1, dadurch gekennzeichnet, daß das oder die Öle gewählt werden aus der Gruppe Sojaöl, Sonnenblumenöl, Weizenkeimöl, Rizinusöl.

10. Verwendung nach Anspruch 1, dadurch gekennzeichnet, daß sie 0 bis 60 Gew.-% Sojaöl und 0 bis 60 Gew.-% Weizenkeimöl und 0 bis 60 Gew.-% Sonnenblumenöl enthalten, mit der Maßgabe, daß die Summe der Einzelkonzentrationen dieser Öle 30 - 60 Gew.-% ausmacht, ferner Rizinusöl in einer Konzentration von 5 - 25 Gew.-%, wobei diese Konzentrationen jeweils bezogen sind auf das Gesamtgewicht der Zubereitungen.

## Claims

1. Use of compositions with
(a) a content of not more than 55% by weight, based on the total weight of the formulations, of one or more surfactants selected from the group of fatty alcohol ethoxylates, fatty alcohol sulphates, amides of fatty alcohol sulphates, fatty alcohol ether sulphates, amides of fatty alcohol ether sulphates, fatty acid monoethanolamides, fatty acid diethanolamides, and
(b) a content of not less than 45% by weight, based on the total weight of the formulations, of one or more oil components selected from the group of oils with a high content of triglycerides of saturated and/or unsaturated, branched and/or unbranched fatty acids or containing exclusively such triglycerides,
(c) which are moreover essentially anhydrous,
for producing cosmetic or dermatological shower oils.

2. Use according to Claim 1, characterized in that additionally further surfactants and/or cosmetic or pharmaceutical auxiliaries, additives and/or active substances, which correspondingly reduce the content of (a), are present.

3. Use according to Claim 1, characterized in that the fatty alcohol sulphates and fatty alcohol ether sulphates have the following structure: 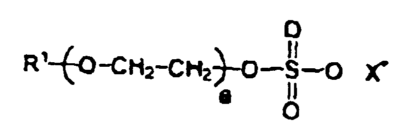 where a assumes values from 0 to 10, advantageously 1 to 5, and R¹ is selected from the group of branched and unbranched alkyl groups with 6 to 24 carbon atoms and X⁺ is selected from the group of alkali metal ions and from the group of ammonium ions which are substituted by one or more alkyl and/or by one or more hydroxyalkyl radicals.

4. Use according to Claim 1, characterized in that the amides of the fatty alcohol sulphates and of the fatty alcohol ether sulphates have the following structure: 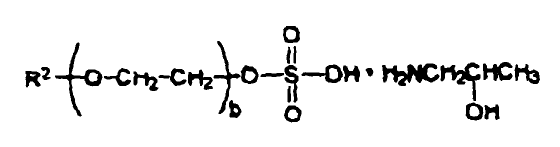 where b assumes values from 0 to 10, advantageously 1 to 5, and R² is selected from the group of branched and unbranched alkyl groups with 6 to 24 carbon atoms.

5. Use according to Claim 1, characterized in that the fatty alcohol ethoxylates have the following structure: 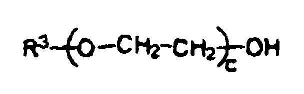 where c assumes values from 1 to 45, preferably from 1 to 10, and R³ is selected from the group of branched and unbranched alkyl groups with 6 to 24 carbon atoms.

6. Use according to Claim 1, characterized in that the fatty acid mono- and diethanolamides have the following structures: 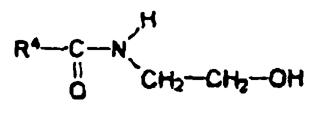 or 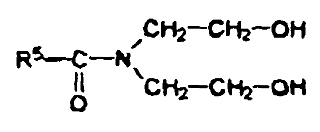 where R⁴ and R⁵ are selected from the group of branched and unbranched alkyl groups and/or alkenyl groups with 6 to 24 carbon atoms.

7. Use according to Claim 1, characterized in that the oils are selected from the group of triglycerides of the following structure: 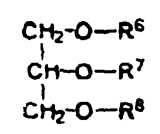 where R⁶, R⁷ and R⁸ are, independently of one another, selected from the group of branched and unbranched alkylcarboxyl and alkenylcarboxyl groups with 12 to 24 carbon atoms and, where appropriate, one or more hydrogen atoms of the alkylcarboxyl and alkenylcarboxyl groups are replaced by hydroxyl groups.

8. Use according to Claim 7, characterized in that R⁶, R⁷ and/or R⁸ have 16 to 20 carbon atoms and are selected from the group of mono- to triunsaturated carboxylic acid residues.

9. Use according to Claim 1, characterized in that the oil or oils are selected from the group of soya oil, sunflower oil, wheatgerm oil and castor oil.

10. Use according to Claim 1, characterized in that they contain 0 to 60% by weight of soya oil and 0 to 60% by weight of wheatgerm oil and 0 to 60% by weight of sunflower oil with the proviso that the total of the individual concentrations of these oils amounts to 30-60% by weight, furthermore castor oil in a concentration of 5-25% by weight, where these concentrations are in each case based on the total weight of the formulations.

## Revendications

1. Utilisation de compositions comportant
(a) une teneur d'au maximum 55% en poids, par rapport au poids total des préparations, en un ou plusieurs tensio-actifs, choisis dans l'ensemble constitué par des éthoxylates d'alcools gras, des sulfates d'alcools gras, des amides des sulfates d'alcools gras, des éther-sulfates d'alcools gras, des amides des éther-sulfates d'alcools gras, des monoéthanolamides d'acides gras, des diéthanolamides d'acides gras, ainsi que
(b) une teneur d'au moins 45% en poids, par rapport au poids total des préparations, en un ou plusieurs composants huileux, choisis dans le groupe des huiles ayant une forte teneur en triglycérides d'acides gras saturés et/ou insaturés, ramifiés et/ou non ramifiés ou contenant exclusivement de tels glycérides,
(c) qui en outre sont pratiquement anhydres,
pour la préparation d'huiles de douche cosmétiques ou dermatologiques.

2. Utilisation selon la revendication 1, caractérisée en ce que sont en outre contenus d'autres tensio-actifs et/ou des adjuvants, additifs et/ou substances actives, cosmétiques ou pharmacologiques, qui réduisent de façon appropriée la teneur en (a).

3. Utilisation selon la revendication 1, caractérisée en ce que les sulfates d'alcools gras ou éther-sulfates d'alcools gras présentent la structure suivante : 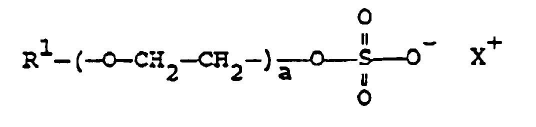 dans laquelle a prend des valeurs de 0 à 10, avantageusement de 1 à 5, et R¹ est choisi dans l'ensemble des groupes alkyle ramifiés et non ramifiés ayant de 6 à 24 atomes de carbone, et X⁺ est choisi dans le groupe des ions alcalins ainsi que dans le groupe des ions ammonium substitués par un ou plusieurs radicaux alkyle et/ou par un ou plusieurs radicaux hydroxyalkyle.

4. Utilisation selon la revendication 1, caractérisée en ce que les amides des sulfates d'alcools gras ou des éther-sulfates d'alcools gras présentent la structure suivante : 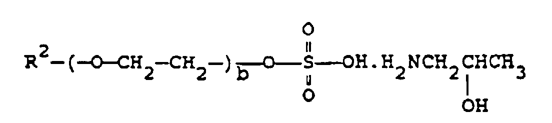 dans laquelle b prend des valeurs de 0 à 10, avantageusement de 1 à 5, et R² est choisi dans l'ensemble des groupes alkyle ramifiés et non ramifiés ayant de 6 à 24 atomes de carbone.

5. Utilisation selon la revendication 1, caractérisée en ce que les éthoxylates d'alcools gras présentent la strucutre suivante :
R³-(-O-CH₂-CH₂-)_{c}-OH
dans laquelle c prend des valeurs de 1 à 45, de préférence de 1 à 10, et R³ est choisi dans l'ensemble des groupes alkyle ramifiés et non ramifiés ayant de 6 à 24 atomes de carbone.

6. Utilisation selon la revendication 1, caractérisée en ce que les mono- ou diéthanolamides d'acides gras présentent les structures suivantes : 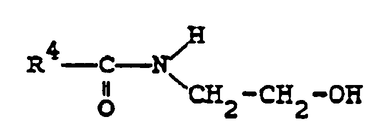 ou 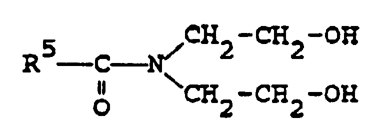 dans lesquelles R⁴ et R⁵ sont choisis dans l'ensemble des groupes alkyle et/ou alcényle ramifiés et non ramifiés ayant de 6 à 24 atomes de carbone.

7. Utilisation selon la revendication 1, caractérisée en ce que les huiles sont choisies dans le groupe des triglycérides de structure suivante : 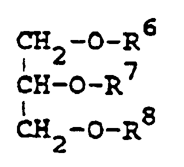 dans laquelle R⁶, R⁷ et R⁸ sont choisis, indépendamment les uns des autres, dans l'ensemble des groupes alkylcarboxyle ou alcénylcarboxyle ramifiés et non ramifiés, ayant de 12 à 24 atomes de carbone, et éventuellement un ou plusieurs atomes d'hydrogène des groupes alkylcarboxyle ou alcénylcarboxyle est (sont) remplacé(s) par un (des) groupe(s) hydroxyle.

8. Utilisation selon la revendication 7, caractérisée en ce que R⁶, R⁷ et/ou R⁸ comportent de 16 à 20 atomes de carbone et sont choisis dans le groupe des radicaux acides carboxyliques ayant une à trois insaturations.

9. Utilisation selon la revendication 1, caractérisée en ce que la ou les huile(s) sont choisie(s) dans le groupe constitué par l'huile de soja, l'huile de tournesol, l'huile de germes de blé, l'huile de ricin.

10. Utilisation selon la revendication 1, caractérisée en ce que les compositions contiennent de 0 à 60% en poids d'huile de soja et de 0 à 60% en poids d'huile de germes de blé et de 0 à 60% en poids d'huile de tournesol, étant entendu que la somme des concentrations individuelles de ces huiles représente de 30 à 60% en poids, et en outre de l'huile de ricin à une concentration de 5 à 25% en poids, ces concentrations étant dans chaque cas par rapport au poids total des préparations.
